# EUROPÄISCHE PATENTSCHRIFT

(11) **EP 0 884 985 B1**
(45) Veröffentlichungstag und Bekanntmachung des Hinweises auf die Patenterteilung: **25.06.2003**
(21) Anmeldenummer: 97947723.9
(22) Anmeldetag: 28.10.1997
(51) Int. Cl.: A61F 2/06

(54) **STENT**
STENT
EXTENSEUR

(30) Priorität: 28.10.1996 DE 19645288; 10.12.1996 DE 19653718
(43) Veröffentlichungstag der Anmeldung: 23.12.1998
(73) Patentinhaber: BIOTRONIK Mess- und Therapiegeräte GmbH & Co Ingenieurbüro Berlin, 12359 Berlin (DE)
(72) Erfinder: KRANZ, Curt, D-10825 Berlin (DE)
(74) Vertreter: Christiansen, Henning, Dipl.-Ing.
(86) Internationale Anmeldenummer: DE9702576
(87) Internationale Veröffentlichungsnummer: WO98018406

(56) Entgegenhaltungen:
- EP-A- 0 364 787
- US-A- 5 449 373

## Beschreibung

Die Erfindung betrifft einen Stent, insbesondere Koronarstent, als intraluminales Expansionselement, entsprechend der im Oberbegriff des Anspruchs 1 genannten Art.

Aus den europäischen Patentschriften EP-B1 0 364 787 und EP-B1 335 341 ist ein aufweitbares intraluminales Element mit mindestens einem dünnwandigen, rohrförmigen Teil (nachfolgend als Stent bezeichnet) bekannt. Die Mantelfläche des Stents ist durchbrochen netzförmig ausgebildet und weist dabei Ausnehmungen auf, die durch sich geradlinig in axialer und in Umfangsrichtung erstreckende stegartigen Elemente von geringer Materialstärke begrenzt sind. Die stegartigen Elemente bestehen aus der restlichen Rohrwandung, von der das Material im Bereich der Ausnehmungen entfernt wurde.

Derartige Stents werden in einem oprerativen Eingriff unter Einwirkung von von innen nach außen gerichteten Kräften durch einen mit Druckgas beaufschlagten schlauchförmigen Dilator, expandiert. Der Stent behält dabei trotz Verformung seine Rohrform bei und weitet das durch Ablagerungen verengte Gefäß auf.

Der bekannte Stent weist den Nachteil auf, daß das Expandieren aufgrund der Verformung der sich axial erstreckenden stegartiges Elemente nur in beschränktem Maße erfolgen kann, da der Formänderung der einzelnen stegartigen Elemente des Stents relativ enge Grenzen gesetzt sind. Diese Grenzen sind bedingt durch die mit der Verformung einhergehende Materialspannungen, welche - wenn die Verformung zu stark wird - zum Bruch von einem oder mehreren der das Netz bildenden stegartigen Elemente können,

Aus Sicherheitsgründen muß die Verformung deshalb normalerweise weit unterhalb eines möglichen Gefahrenbereichs gehalten werden, da der Bruch eines Steges dazu führen würde, daß dessen freie Enden im Bereich der Bruchstelle in das Innere des mit dem Stent versehenen Gefäßes hineinragen würde. Durch die damit verbundene Gefahr der Bildung von Restenosen würde nicht nur der Erfolg des Eingriffs selbst infrage gestellt, sondern auch das Leben des Patienten gefährdet.

Ausgehend von den Mängeln des Standes der Technik liegt der Erfindung die Aufgabe zugrunde, einen expandierbaren Stent der eingangs genannten Gattung anzugeben, welcher möglichst sicher - und damit auch ohne die Gefahr eines durch Spannungsüberlastung bedingten Bruchs im Bereich der stegartiges Elemente, expandierbar ist. Außerdem soll auch eine Verkürzung des Stents vermieden werden.

Die Aufgabe wird durch die kennzeichnenden Merkmale des Anspruchs 1 gelöst.

Die Erfindung schließt die technische Lehre ein, daß bei fragilen aus geeigneten Werkstoffen gefertigten, netzartige Strukturen aufweisenden rohrförmige Elementen, welche anwendungsbedingt einer Verformung unterzogen werden, kritische Materialbelastungen oder gar Materialbrüche vermeidbar sind, wenn in solchen Bereichen, die einer erhöhten Verformung unterworfen sind, bereits konstruktiv und von vorn herein die auftretenden Spannungsmaxima begrenzt werden. Wenn darüberhinaus in Längsrichtung des Stents jeweils zwei in Querrichtung expansible Elemente stirnseitig unmittelbar miteinander verbunden sind, wobei jedes dieser beiden expansiblen Elemente, in Form eines aus Stegen gebildeten gestauchten Ringelements, in Querrichtung über je ein stegartiges Element mit jeweils einem weiteren in Querrichtung expansiblen Element verbunden ist, welches seinerseits nicht mit einem anderen expansiblen Element stirnseitig unmittelbar verbunden ist, das wiederum mit einem der erstgenannten in Querrichtung expansiblen Elemente mit einem stegartiges Element verbunden ist, wobei die stegartigen Elemente jeweils in einem solchen Winkel zur Querrichtung geneigt verlaufen, daß dieser Winkel sich bei Expansion des Stents verringert und sich somit die nicht verbundenen benachbarten stirnseitigen Enden von in Querrichtung expandierbaren Elementen voneinander entfernen können auch Verkürzungen des Stents bei dessen Expansion vermieden werden.

Dies gilt insbesondere, wenn die stegartigen Elemente bei nicht expandiertem Stent eine Neigung zur Querrichtung von im wesentlichen 45° aufweisen.

Durch den bei der Expansion des Stents gleichzeitig auftretenden Vorgang der Dehnung der expandierbaren Element in Querrichung des Stents und durch das Ausrichten der schrägstehenden Verbindungselement in Querrichtung werden die jeweils nicht miteinander verbundenen Gruppen von expansiblen Elementen so gegeneinander verschoben, daß diese Bewegung die Verkürzung des Stents durch Ausdehnung der flachen Formen der expansiblen Elemente zu O-Ringen kompensiert.

Die in Querrichtung expansiblen Element wirken dabei besonders günstig mit den stegartigen Elementen zusammen, wenn diese in die in Querrichtung expandierbaren Elemente - bezogen auf deren lokale Richtung - unter einem Winkel von weniger als 45° einmünden.

Wenn die stegartigen Elemente gebogen sind, um senkrecht mittig in die Stegbereiche der expandierbaren Element einzumünden ergibt sich eine besonders günstige Einleitung der Kräfte.

Durch die Geometrie der Anordnung in nicht expandiertem Zustand bedingt, ist es günstig, wenn die Verrundung der in Querrichtung expandierbaren Elemente in dem Endbereich mit dem sie stirnseitig mit einem in Längsrichtig folgenden gleichartigen Element verbunden sind, einen größeren Radius aufweist als an dem Ende, an dem sie unverbunden sind.

Wenn die in Querrichtung expandierbaren Elemente in expandiertem Zustand des Stents eine ellipsen- oder kreisähnliche Form aufweisen, ergibt sich eine besonders harmonische Krafteinleitung in das aufgeweitete Gefäß.

Dies gilt auch, wenn die stegartigen Elemente im bei expandiertem Stent im wesentlichen an einander in Querrichtung gegenüberliegenden Bereichen des in Querrichtung expandierbaren Elements angreifen - und insbesondere im wesentlichen mittig angreifen.

Eine besonders günstige Konstruktion ergibt sich noch nicht nur durch die konstruktive Auslegung in Bezug auf eine möglichst große Festigkeit durch Erhöhung der Materialquerschnitte, sondern auch dadurch, daß die Form der Stege und Verbindungsbereiche im Hinblick auf die zu erwartenden Belastungen optimiert wird. Dies geschieht einerseits dadurch, daß die auftretenden maximalen Spannungen lokal minimiert werden, anderseits aber auch dadurch, daß die notwendigen Verformungen kontrolliert werden.

Diese Zusammenhänge sind in einer gleichzeitig eingereichten Patentanmelderin derselben Inhaberin beschrieben.

Als vorteilhafte Konstruktionsvoraussetzung wurde gefunden, daß die Verformung begünstigt ist, wenn die Form des Stents in nicht expandiertem Zustand im wesentlichen derjenigen Form entspricht, die sich ergibt, wenn ein in expandierter Form Stegstrukturen von regelmäßiger Form aufweisendes, in dieser Form aus einer rohrförmigen Struktur erstelltes Muster, in die nicht expandierte Form - die spätere Ausgangsform - komprimiert wird. Damit wird also eine Form als Ausgangsform gefertigt, die derjenigen enspricht, die sich ergibt, wenn man einen in seiner expandierten Form gefertigten Stent komprimiert.

Derartige regelmäßige Formen werden bevorzugt aus Kreisen, Ellipsen, Rechtecken, Quadraten, Vielecken oder aus diesen zusammengesetzten bzw. an diese angenäherten Gebilden erzeugt.

Um lokale spannungsfreie Verformungen lokal zu begünstigen, ist es vorgesehen, daß Verzweigungen von Stegen unter Vermeidung von sprungartigen Änderungen der Stegbreite erzeugt sind. Damit wird das entstehen von Kerbspannungen und dergleichen vermieden. Hiermit läßt sich erreichen, daß die Materialspannungen insbesondere im Bereich von Kreuzungen oder Verzweigungen bei der Verformung auch als Kerbspannung einen vorgegebenen Wert nicht überschreitet.

Eine derartige Konstruktion führt zu dazu, daß die Verzweigungen von stegartiges Elementen eine besonders organische Form aufweisen, welche im wesentlichen der Form von Stammverzweigungen bei Bäumen entspricht.

In einigen Fällen ist es günstig, wenn die stegartigen Elemente als Verbindungen zwischen expandierbaren Elementen im wesentlichen S-förmig ausgebildet sind, da dann Bereiche maximaler Verformungen von den Einmündungs- oder Verbindungsbereichen in die freien Stegbereiche verlegt werden.

Vorteilhaft ist es weiterhin, wenn auch Bereiche von Kreuzungen derart geformt sind, daß für eine vorgebenen Expansion eine maximale Änderung des Winkels zwischen benachbarten stegartiges Elementen als Armen eines Kreuzes oder von Verzweigungen nicht überschritten wird. Dies erfolgt bevorzugt in der Wiese, daß stegartigen Elemente als Arme eines Kreuzes oder von Verzweigungen bogenförmig ausgebildet sind.

Wenn der Stent die Kanten von stegartigen Elementen in einem Kreuzungs- oder Verzweigungsbereich oder im Bereich eines aufeinanderfolgende Stentsegmente verbindenden Verbindungsbereiches Verrundungen aufweisen, daß scharfe Ecken vermieden sind, beiben hier die maximalen Spannungen begrenzt.

Entsprechend einer bevorzugten Ausführungsform der Erfindung weist der expandierbare, im wesentlichen hohlzylindrisch ausgebildete Stent mit einer durch Ausnehmungen, netzartig strukturierten Mantelfläche an den Verbindungspunkten der die Ausnehmungen begrenzenden stegartiges Elemente eine organische Formgebung auf, um hier eine hohe, gegebenenfalls zum Bruch führende Kerbspannung auszuschließen. Ein örtliches Aufbrechen des Stents führt in nachteiliger Weise zu freien, relativ scharfkantigen Enden innerhalb der Raumkonfiguration des Stent, welche einerseits die Gefäßwandung durchbohren können oder andererseits den freien Querschnitt des Gefäßes durch ein Auslenken in die Blutbahn reduzieren.

Diese Formgebung ist durch eine Verrundung aller Verbindungspunkte der sich beim Expandieren des Stents relativ zueinander bewegender stegartiges Elemente gekennzeichnet und sichert in einfacher und zugleich vorteilhafter Weise, daß das Maß der lokalen Materialverformung an den hinsichtlich der auftretenden Spannungsbelastung kritischen Punkten der Stentkonstruktion beim Aufweiten des Stents durch gleichmäßige Verteilung der Verformungsarbeit einen minimalen Wert aufweist.

Zur Verbindung der einzelnen, durch sich im wesentlichen axial erstreckenden und die Ausnehmungen begrenzenden stegartiges Elemente sind als kreuzförmiger stegartiges Element ausgebildete Koppelglieder vorgesehen, welche die benachbart angeordneten Ausnehmungen in axialer Richtung jeweils an ihren Enden und in tangentialer Richtung jeweils in Mitte der sich axial erstreckenden stegartiges Elemente miteinander verbinden.

Nach einer bevorzugten Ausführungsform der Erfindung sind die Arme der kreuzförmigen Koppelglieder bogenförmig ausgebildet und derart angeordnet, daß sie in tangentialer Richtung die Schenkel eines im wesentlichen stumpfen Winkels und in axialer Richtung einen im wesentlichen spitzen Winkel bilden. Die sich an die Arme der Koppelglieder anschließenden stegartiges Elemente der Ausnehmungen sind als in axialer Richtung gestrecktes S ausgebildet, wobei die in tangentialer Richtung einander benachbart gegenüberliegenden stegartiges Elemente spiegelsymmetrisch angeordnet sind.

Bei dieser Konfiguration des Koppelgliedes sind nach einer anderen Weiterbildung der Erfindung in vorteilhafter Weise im Kreuzungspunkt Materialverrundungen vorgesehen, so daß bei Expandieren des Stents nur eine relativ geringfügige Veränderung der zwischen den benachbarten Armen des kreuzförmigen Koppelgliedes eingeschlossenen Winkel eintritt. Dadurch bedingt treten auch keine hohe Kerbspannung im Kreuzungspunkt, insbesondere keine lokale Spannungsspitzen, auf, so daß eine Bruchgefahr beim Expandieren ausgeschlossen ist.

Unter dem gleichen Gesichtspunkt sind die an den Stentenden bzw. an den Enden der Segmente des Stents der vorstehend beschriebenen Ausführungsformen der Erfindung befindlichen freien Abschnitte der Ausnehmungen bogenförmig ausgebildet, so daß auch in diesen Bereichen beim Expandieren des Stents nur geringfügige mechanische Spannungen entstehen und keine Kerbspannungs-Extremwerte auftreten.

Die vorstehend beschriebene, organische Formen aufweisende Ausbildung der Mantelfläche des erfindungsgemäßen Stents sichert eine im wesentlichen gleichmäßige Verteilung der beim Expandieren geleisteten Verformungsarbeit auf die jeweilige Ausnehmungen begrenzenden Abschnitte und vermeidet dadurch extreme Spannungsbelastungen einzelner Punkte oder Bereiche auf der Mantelfläche des Stents.

Um auch in gekrümmten Blutgefäßen vorhandene Stenosen erfolgreich durch das Expandieren von Stents therapieren zu können, ist die Stent entsprechend einer anderen günstigen Ausführungsform der Erfindung in im wesentlichen gleichartig ausgebildete, in axialer Richtung reihenförmig angeordnete Segmente gegliedert.

Ein bevorzugte Stent der vorstehnden Auslegung besteht aus Tantal als Werkstoff und ist mit einer Beschichtung aus amorphen Siliciumcarbid versehen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 als bevorzugte Ausführungsform eine Abwicklung des nicht expandierten Stentstruktur, dargestellt als Abwicklung der Erfindung in Seitenansicht,
Figur 2 die Stentstruktur nach Figur 1 in expandiertem Zustand sowie
Figur 3 die Zustände gemäß den Figuren 1 und 2 zum Vergleich überlagert.

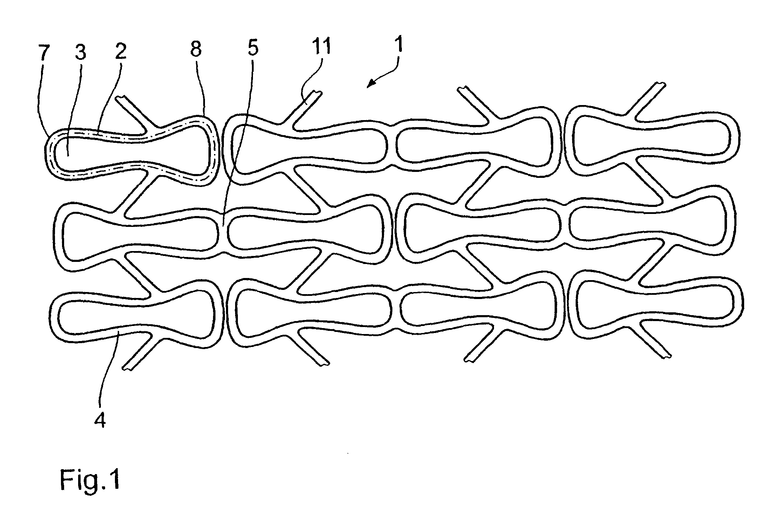

Ein in Figur 1 wiedergegebener Stent 1 weist eine rohrförmig/hohlzylindrische Grundform mit zahlreichen Durchbrüchen auf, welche von expansiblen Strukturelementen umschlossen sind, die die Form von gestauchten Ringen aufweisen. Diese werden nachfolgend als "expansible Elemente" bezeichnet und sind an einem Beispiel mit einer strichpunktierten Linie 2 markiert. Diese expansiblen Elemente 2 werden gebildet durch umlaufende schmale stegartige Bereiche 4 mit rechtekkigem Querschnitt und zeichnen sich dadurch aus, daß sie eine Ausnehmung 3 ringförmig umschließen. Die expansiblen Elemente 2 haben in diesem Fall in vollständig expandiertem Zustand nahezu die Form eines Kreises oder einer Ellipse. Es ist ersichtlich, daß die Form im nicht expandierten Zustand aus der Form des expandierten Zustands abgeleitet ist, obwohl der Stent, wenn er hergestellt ist, diesen Zustand nie eingenommen hat. Die Form wurde gefunden, indem in einem simulierten Verfahren - ausgehend einer im expandierten Zustand einzunehmenden Idealform - die Kompression in einer Modellrechnung simuliert wurde. Da das Material bei der Kompression den dazu angreifenden mechanischen Kräften gleichmäßig Widerstand entgegensetzt, sind die lokalen Verformungen ebenfalls vergleichmäßigt. Es entstehen keine lokalen starken Abknickungen, sondern gleichmäßige Bögen, deren Radien maximal sind. Die so erhaltene Form wird dem Entwurf der nicht expandierten Form zugrundegelegt, welche sich in umgekehrter Richtung auf diese Weise unter gleichmäßiger lokaler Verformung in die angestrebte Endform überführen läßt.

Die ein expansibles Element umschließenden stegartigen Bereiche 4 sind dabei mehrfach S-förmig geschwungen ausgebildet. Sie umschließen jeweils eine Ausnehmung 3 in der Weise, daß die sich in tangentialer Richtung benachbart gegenüberliegenden stegartiges Bereiche 4 der selben oder einer benachbarten Ausnehmung 3 spiegelsymmetrisch angeordnet sind. An ihren in Längsrichtung gelegenen Enden weisen die expansiblen Elemente 2 freie Bogenbereiche 7 und 8 mit vergrößerter Krümmung auf.

Die expansiblen Elemente sind derart geformt, daß sie sich nach dem Einbringen des Stents in ein Gefäß durch Dilatieren mit einem Ballonkatheter mit minimaler Verformung in eine Ringform umwandeln. Es ist ersichtlich, daß dabei der Bogen 8 einen maximalen Radius einnimmt. Durch die S-förmigen Gegenbögen ist es ihm ermöglicht, bei der Expansion eine möglichst geringe Verformung zu erleiden.

Zwischen den in axialer (Längs-) und in tangentialer (Quer) Richtung benachbart auf der Mantelfläche des Stents 1 angeordneten Ausnehmungen 3 sind Verbindungsbereiche 5 vorgesehen, welche die jeweiligen expandierbaren Bereiche 2 mechanisch miteinander koppeln. Im kreuzenden Verbindungsbereich 5 sind dabei Materialverrundungen derart vorgesehen, so daß der Verbindungsbereich eine organische, das Auftreten von erhöhten Kerbspannungen vermindernde Form aufweist.

Es ist ersichtlich, daß die einzelnen stegartigen Elemente derart geformt sind, daß die Biegeverformung, der ein stegartiges Element bei der Expansion innerhalb der hohlzylindrischen Rohrform unterworfen ist und die sich ergibt aus dem Integral der örtlichen Winkeländerungen bei Verformung, ermittelt über die Länge des jeweiligen stegartigen Elements zwischen angrenzenden Verbindungsbereichen mit anderen stegartigen Elementen sich in der Weise über die Länge des Elements verteilt, so daß auch lokal einen vorgegebe Materialbeanspruchung nicht überschritten wird.

Die Form des Stents entspricht in nicht expandiertem Zustand im wesentlichen derjenigen Form, die sich ergibt, wenn ein in expandierter Form Stegstrukturen von regelmäßiger Form aufweisendes, in dieser Form aus einer rohrförmigen Struktur erstelltes Muster, in die nicht expandierte Form - die spätere Ausgangsform - komprimiert wird. Die regelmäßige Form besteht aus Kreisen, Ellipsen, Rechtecken, Quadraten, Vielecken oder aus diesen zusammengesetzten bzw. an diese angenäherten Gebilden.

Verzweigungen von Stegen sind unter Vermeidung von sprungartigen Änderungen der Stegbreite derart gestaltet, daß die Materialspannungen insbesondere im Bereich der Verzweigung bei der Verformung auch als Kerbspannung einen vorgegebenen Wert nicht überschreitet. Der in Figur la wiedergegebene Stent ist über seine gesamte Länge im wesentlichen homogen strukturiert.

Es ist ersichtlich, daß bei der dargestellten Ausführungsform in Längsrichtung des Stents jeweils zwei in Querrichtung expansible Elemente stirnseitig unmittelbar miteinander verbunden sind, wobei jedes dieser beiden expansiblen Elemente, in Form eines aus Stegen gebildeten gestauchten Ringelements, in Querrichtung über je ein stegartiges Element mit jeweils einem weiteren in Querrichtung expansiblen Element verbunden ist, welches seinerseits nicht mit einem anderen expansiblen Element stirnseitig unmittelbar verbunden ist, das wiederum mit einem der erstgenannten in Querrichtung expansiblen Elemente mit einem stegartiges Element verbunden ist, wobei die stegartigen Elemente jeweils in einem solchen Winkel zur Querrichtung geneigt verlaufen, daß dieser Winkel sich bei Expansion des Stents verringert und sich somit die nicht verbundenen benachbarten stirnseitigen Enden von in Querrichtung expandierbaren Elementen voneinander entfernen können auch Verkürzungen des Stents bei dessen Expansion vermieden werden.

Die stegartigen Elemente weisen bei nicht expandiertem Stent eine Neigung zur Querrichtung von im wesentlichen 45° auf.

Durch den bei der Expansion des Stents gleichzeitig auftretenden Vorgang der Dehnung der expandierbaren Element in Querrichung des Stents und durch das Ausrichten der schrägstehenden Verbindungselement in Querrichtung werden die jeweils nicht miteinander verbundenen Gruppen von expansiblen Elementen so gegeneinander verschoben, daß diese Bewegung die Verkürzung des Stents durch Ausdehnung der flachen Formen der expansiblen Elemente zu O-Ringen kompensiert.

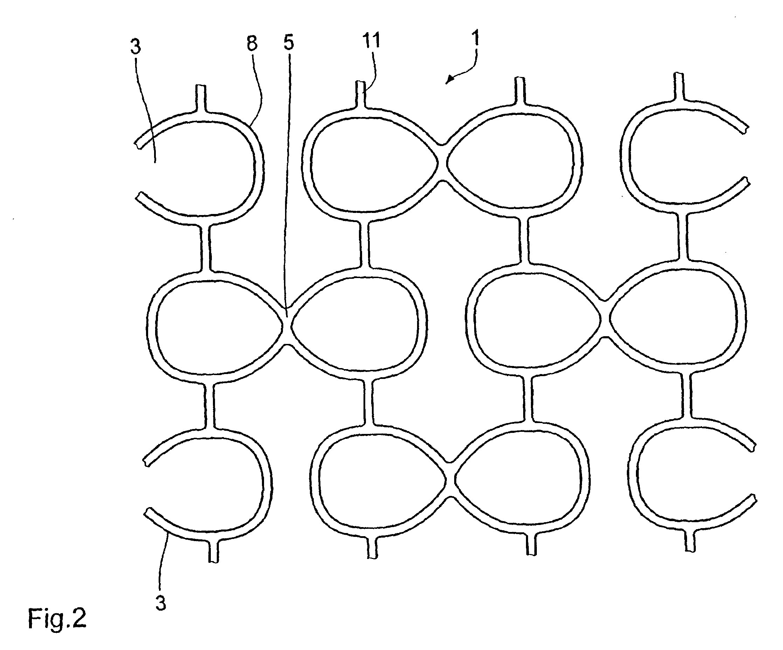

In Figur 2 ist - unter Verwendung derselben Bezugszeichen wie in Figur 1 - ersichtlich, wie die Struktur gemäß Figur sich in expandiertem Zustand zu einer Struktur mit weitgehend runden Ringen formt, die mit Stegen untereinander verbunden sind. Die Verbindungsstege zwischen den expansiblen Elementen sind jetzt weitgehend tangential ausgerichtet.

Es ist auch ersichtlich, daß die einzelnen stegartigen Elemente derart geformt sind, daß die Biegeverformung, der ein stegartiges Element bei der Expansion innerhalb der hohlzylindrischen Rohrform unterworfen ist und die sich ergibt aus dem Integral der örtlichen Winkeländerungen bei Verformung, ermittelt über die Länge des jeweiligen stegartigen Elements zwischen angrenzenden Verbindungsbereichen mit anderen stegartigen Elementen sich in der Weise über die Länge des Elements verteilt, daß sie lokal einen vorgegebenen Wert nicht überschreitet.

Es ist ferner erkennbar, daß Verzweigungen von Stegen unter Vermeidung von sprungartigen Änderungen der Stegbreite ausgebildet sind, und daß Verzweigungen derart ausgebildet sind, daß die Materialspannungen insbesondere im Bereich der Verzweigung bei der Verformung auch als Kerbspannung einen vorgegebenen Wert nicht überschreitet.

Die vorstehend beschriebene Ausbildung der Stents 1 gestattet ein Expandieren der rohrförmigen Stents, ohne daß es an den Verbindungspunkten zur Ausbildung von zur Zerstörung von Stegbereichen führenden Extremwerten der Kerbspannung kommt.

Wie in Figur 2 ersichtlich, bleiben die maximalen örtlichen Verformungen - damit auch die Gefahr der Ausbildung von Extremwerten der Kerbspannung beim Expandieren des Segmentes 2 - äußerst gering. Sie konzentrieren sich nicht auf einzelnen Punkte der einzelnen Ausnehmungen 3, insbesondere auf die Spitze der äußeren Bogenstücke 7, sondern erstrekken sich über den gesamten Bereich der durch die S-förmigen stegartiges Elemente 4 und die Arme 5.1, 5.2, 5.3, 5.4 der Verbindungsbereiche 5 begrenzten Ausnehmungen 3.

Es ist vor allen Dingen auch ersichtlich, daß die Verformung eines durch Stegelemente gebildeten expansiblen Elements so erfolgt, daß die örtlichen Verformungen möglichst begrenzt bleiben. Die Bogenbereiche in nicht expandiertem Zustand werden möglichst groß gewählt, so daß bei der Expansion alle Teile der stegartigen Elemente möglichst gleichmäßig an der Umformung beteiligt sind.

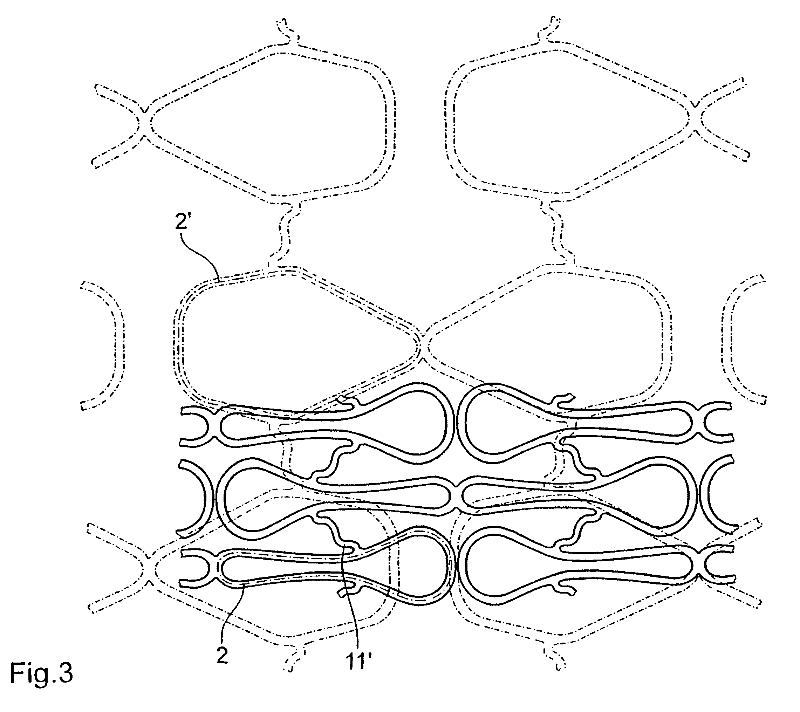

Aus Figur 3, in der die Struktur des nicht expandierten Stents gemäß Figur 1 und des expandierten Stents gemäß Figur 2 superponiert sind, ist erkennbar, nicht nur keine Verkürzung, sondern sogar eine gewisse Längung des Stents eintritt, die jedoch bei weiterer Expansion wieder dahingehend kompensiert wird, daß die Länge des Stents wieder der Ausgangslänge entspricht. Damit ist eine weitestgehend komplikationsfreie Anwendung gesichert. Das stegartige Verbidnungselement 11' ist bei dem in dieser Figur dargestellten Ausführungsbeispiel doppelt s-förmig gekrümmt ausgebildet, um die Verformungen zu minimieren.

Der hier dargestellte Stent besteht aus Tantal, Titan oder einer anderen biokompatiblen Materiallegierung als Werkstoff, woraus eine gute Körperverträglichkeit und eine ausgezeichnete Verformbarkeit resultiert. Eine Mikrobeschichtung aus amorphem Siliciumcarbid wirkt einer Thrombenbildung entgegen.

Die Erfindung beschränkt sich in ihrer Ausführung nicht auf die vorstehend angegebenen bevorzugten Ausführungsbeispiele. Vielmehr ist eine Anzahl von Varianten günstig, welche von der dargestellten Lösung auch bei grundsätzlich anders gearteten Ausführungen Gebrauch macht.

## Patentansprüche

1. Stent (1), insbesondere Koronarstent, bestehend aus mindestens einem dünnwandigen, rohrförmigen Element, dessen Mantelfläche durchbrochen netzförmig ausgebildet ist und dabei Ausnehmungen (3) aufweist, die durch stegartige Elemente (4) von geringer Breite begrenzt sind, wobei die stegartigen Elemente (4) aus dem restlichen Material der Rohrwandung gebildet werden, von dem das Material im Bereich der Ausnehmungen (3) entfernt wurde, **dadurch gekennzeichnet, daß** in Längsrichtung des Stents (1) jeweils zwei in Querrichtung expansible Elemente (2) stirnseitig unmittelbar miteinander verbunden sind, wobei jedes dieser beiden expansiblen Elemente, in Form eines aus Stegen gebildeten gestauchten Ringelements, in Querrichtung über je ein stegartiges Element (11) mit jeweils einem weiteren in Querrichtung expansiblen Element (2) verbunden ist, welches seinerseits nicht mit einem anderen expansiblen Element (2) stirnseitig unmittelbar verbunden ist, das wiederum mit einem der erstgenannten in Querrichtung expansiblen Elemente (2) mit einem stegartiges Element (11) verbunden ist, wobei die stegartigen Elemente (11) jeweils in einem solchen Winkel zur Querrichtung geneigt verlaufen, daß dieser Winkel sich bei Expansion des Stents verringert und sich somit die nicht verbundenen benachbarten stirnseitigen Enden von in Querrichtung expandierbaren Elementen (2) voneinander entfernen.

2. Stent nach Anspruch 1, **dadurch gekennzeichnet, daß** die stegartigen Elemente (11) bei nicht expandiertem Stent (1) eine Neigung zur Querrichtung von im wesentlichen 45° aufweisen.

3. Stent nach Anspruch 1, **dadurch gekennzeichnet, daß** die stegartigen Elemente (11) in die in Querrichtung expandierbaren Elemente (2) unter einem Winkel von weniger als 45° einmünden.

4. Stent Anspruch 1, **dadurch gekennzeichnet, daß** die stegartigen Elemente (11) gebogen sind, um senkrecht mittig einzumünden.

5. Stent nach Anspruch 1, **dadurch gekennzeichnet, daß** die Verrundung der in Querrichtung expandierbaren Elemente (2) in dem Endbereich mit dem sie stirnseitig mit einem in Längsrichtig folgenden gleichartigen Element (2) verbunden sind, einen größeren Radius aufweist als an dem Ende, an dem sie unverbunden sind.

6. Stent nach Anspruch 1, **dadurch gekennzeichnet, daß** die in Querrichtung expandierbaren Elemente (2) in expandiertem Zustand des Stents eine ellipsen- oder kreisähnliche Form aufweisen,

7. Stent nach Anspruch 1, **dadurch gekennzeichnet, daß** die stegartigen Elemente (11) bei expandiertem Stent im wesentlichen an einander in Querrichtung gegenüberliegenden Bereichen des in Querrichtung expandierbaren Elements (2) angreifen.

8. Stent nach Anspruch 7, **dadurch gekennzeichnet, daß** die stegartigen Elemente (11) im wesentlichen mittig angreifen.

9. Stent nach Anspruch 1, **dadurch gekennzeichnet, daß** die stegartigen Elemente (2) derart geformt sind, daß die Verformung, welcher ein stegartiges Element zwischen Verbindungsbereichen mit anderen stegartigen Elementen (2) bei der Expansion insgesamt unterworfen wird, im wesentlichen minimiert ist.

10. Stent nach Anspruch 1, **dadurch gekennzeichnet, daß** die einzelnen stegartigen Elemente (2) derart geformt sind, daß die Biegeverformung, der ein stegartiges Element (2) bei der Expansion innerhalb der hohlzylindrischen Rohrform unterworfen ist und die sich ergibt aus dem Integral der örtlichen Winkeländerungen bei Verformung, ermittelt über die Länge des jeweiligen stegartigen Elements zwischen angrenzenden Verbindungsbereichen (5) mit anderen stegartigen Elementen (2), sich in der Weise über die Länge des Elements verteilt, daß sie lokal einen vorgegebenen Wert nicht überschreitet.

11. Stent nach Anspruch 1, **dadurch gekennzeichnet, daß** die auf eine Länge von einem Fünftel eines stegartigen Elements (2) entfallende Biegeverformung nicht größer ist als ein Viertel der gesamten Biegeverformung, der dieses stegartige Element (2) unterworfen ist.

12. Stent nach Anspruch 1, **dadurch gekennzeichnet, daß** die Form des Stents (1) in nicht expandiertem Zustand im wesentlichen derjenigen Form entspricht, die sich ergibt, wenn ein in expandierter Form Stegstrukturen von regelmäßiger Form aufweisendes, in dieser Form aus einer rohrförmigen Struktur erstelltes Muster, in die nicht expandierte Form - die spätere Ausgangsform - komprimiert wird.

13. Stent nach Ansprüche 12, **dadurch gekennzeichnet, daß** die regelmäßige Form besteht aus Kreisen, Ellipsen, Rechtecken, Quadraten, Vielecken oder aus diesen zusammengesetzten bzw. an diese angenäherten Gebilden.

14. Stent nach Anspruch 1, **dadurch gekennzeichnet, daß** Verzweigungen von Stegen unter Vermeidung von sprungartigen Änderungen der Stegbreite ausgebildet sind.

15. Stent nach Anspruch 1, **dadurch gekennzeichnet, daß** Verzweigungen derart ausgebildet sind, daß die Materialspannungen insbesondere im Bereich der Verzweigung bei der Verformung auch als Kerbspannung einen vorgegebenen Wert nicht überschreitet.

16. Stent nach Anspruch 1, **dadurch gekennzeichnet, daß** die stegartigen Elemente (11') im wesentlichen S-förmig ausgebildet sind.

17. Stent nach Anspruch 1, **gekennzeichnet durch** Titan, Tantal oder eine anderes biokompatibles Metall bzw. eine entsprechende Metalllegierung als Werkstoff.

18. Stent nach Anspruch 17, **dadurch gekennzeichnet, daß** eine Beschichtung aus amorphem Siliciumcarbid vorgesehen ist.

## Claims

1. Stent (1), in particular a coronary stent, consisting of at least one thin-walled, tubular element, the external surface of which is in the form of an open network and has recesses (3) bounded by narrow, web-like elements (4), the web-like elements (4) being formed from the remaining material of the tubular wall from which the material in the region of the recesses (3) was removed, **characterised in that** pairs of transversely expandable elements (2) are directly joined at the ends longitudinally of the stent (1), each of the two expandable elements, in the form of a flattened ring element made up of webs, being joined transversely by a web-like element (11) to another transversely expandable element (2), which is itself not directly joined at the end to another expandable element (2), which is in turn joined by a web-like element (11) to one of the first-mentioned, transversely expandable elements (2) , the web-like elements (11) each being inclined to the transverse direction at an angle such that this angle is reduced on expansion of the stent, and the non-joined adjacent ends of transversely expandable elements (2) thus move away from each other.

2. Stent according to claim 1, **characterised in that** when the stent (1) is not expanded the web-like elements (11) have an inclination of substantially 45° to the transverse direction.

3. Stent according to claim 1, **characterised in that** the web-like elements (11) lead into the transversely expandable elements (2) at an angle of less than 45°.

4. Stent according to claim 1, **characterised in that** the web-like elements (11) are curved so as to lead in perpendicularly in the centre.

5. Stent according to claim 1, **characterised in that** the rounding of the transversely expandable elements (2) in the end region where they are joined at the end to the next similar element (2) in the longitudinal direction has a larger radius than at the end where they are not joined.

6. Stent according to claim 1, **characterised in that** in the expanded condition of the stent the transversely expandable elements (2) have an ellipse-like or circle-like shape.

7. Stent according to claim 1, **characterised in that** when the stent is expanded the web-like elements (11) substantially act on regions of the transversely expandable element (2) which are opposite each other in the transverse direction.

8. Stent according to claim 7, **characterised in that** the web-like elements (11) exert their action substantially centrally.

9. Stent according to claim 1, **characterised in that** the web-like elements (2) are shaped so that the total deformation which a web-like element undergoes between joining regions to other web-like elements (2) in the expansion process is substantially minimised.

10. Stent according to claim 1, **characterised in that** the individual web-like elements (2) are shaped so that the bending deformation to which a web-like element (2) is subjected on expansion within the hollow cylindrical, tubular shape, resulting from the integral of the local changes of angle on deformation, determined over the length of the respective web-like element between adjoining regions (5) with other web-like elements (2), is distributed over the length of the element so that it does not exceed a predetermined value locally.

11. Stent according to claim 1, **characterised in that** the bending deformation applied to a length of one-fifth of a web-like element (2) is no greater than a quarter of the total bending deformation to which that element (2) is subjected.

12. Stent according to claim 1, **characterised in that** the shape of the stent (1) in the non-expanded condition substantially corresponds to the shape obtained when a sample, which has web structures of regular shape in its expanded shape and which is made **in that** form from a tubular structure, is compressed into the non-expanded shape, the eventual starting shape.

13. Stent according to claim 12, **characterised in that** the regular shape consists of circles, ellipses, rectangles, squares, polygons or structures combining these and/or approximating to them.

14. Stent according to claim 1, **characterised in that** branches from webs are formed while avoiding abrupt changes in the web width.

15. Stent according to claim 1, **characterised in that** branches are formed so that the material tensions, in particular in the region of the branching, do not exceed a predetermined value on deformation, even as a notch tension.

16. Stent according to claim 1, **characterised in that** the web-like elements (11') are substantially S-shaped.

17. Stent according to claim 1, **characterised by** titanium, tantalum or another biocompatible metal or a corresponding metal alloy as the material.

18. Stent according to claim 17, **characterised in that** a coating of amorphous silicon carbide is provided.

## Revendications

1. Extenseur (1), notamment extenseur coronarien, constitué par au moins un élément de forme tubulaire à paroi mince, dont la surface enveloppe est agencée de manière à être perforée à la manière d'un filet et comporte des ouvertures (3) qui sont limitées par des éléments en forme de barrettes (4) ayant une faible largeur, les éléments en forme de barrettes (4) étant formés par le reste de la matière de la paroi tubulaire, dont la matière a été retirée dans la zone des ouvertures (3), **caractérisé en ce que** respectivement deux éléments (2) extensibles dans la direction transversale sont reliés frontalement directement entre eux dans la direction longitudinale de l'extenseur (1), dans lequel chacun de ces deux éléments extensibles, sous la forme d'un élément annulaire comprimé formé par des barrettes, est relié dans la direction transversale par l'intermédiaire d'un élément respectif en forme de barrette (11) à respectivement un autre élément (2) extensible dans la direction transversale, qui pour sa part n'est pas relié directement frontalement à un autre élément extensible (2), qui à nouveau est relié à un élément en forme de barrette (11) au moyen de l'un des éléments (2) indiqué en premier lieu extensible dans la direction transversale, les éléments en forme de barrettes (11) étant respectivement inclinés d'un angle tel par rapport à la direction transversale que cet angle diminue lors de l'extension de l'extenseur et que par conséquent les extrémités frontales voisines, non reliées, d'éléments (2) qui sont extensibles dans la direction transversale, s'écartent l'une de l'autre.

2. Extenseur selon la revendication 1, **caractérisé en ce que** lorsque l'extenseur (1) n'est pas déployé, les éléments en forme de barrettes (11) possèdent une inclinaison égale essentiellement à 45° par rapport à la direction transversale.

3. Extenseur selon la revendication 1, **caractérisé en ce que** les éléments en forme de barrettes (11) rejoignent dans les éléments (2) extensibles dans la direction transversale, sous un angle inférieur à 45°.

4. Extenseur selon la revendication 1, **caractérisé en ce que** les éléments en forme de barrettes (11) sont coudés de manière à se raccorder perpendiculairement en position médiane.

5. Extenseur selon la revendication 1, **caractérisé en ce que** l'arrondi des éléments (2) extensibles dans la direction transversale, dans la zone d'extrémité, avec laquelle ils sont reliés frontalement à l'élément identique (2) suivant dans la direction longitudinale, possède un rayon plus élevé qu'à l'extrémité, au niveau de laquelle les éléments ne sont pas reliés.

6. Extenseur selon la revendication 1, **caractérisé en ce que** les éléments (2) extensibles dans la direction transversale, possèdent une forme similaire à une ellipse ou à un cercle lorsque l'extenseur est à l'état déployé.

7. Extenseur selon la revendication 1, **caractérisé en ce que** lorsque l'extenseur est déployé, les éléments en forme de barrettes (11) attaquent essentiellement des parties, qui sont opposées entre elles dans la direction transversale, de l'élément (2) extensible, dans la direction transversale.

8. Extenseur selon la revendication 7, **caractérisé en ce que** les éléments en forme de barrettes (11) sont connectés essentiellement en position médiane.

9. Extenseur selon la revendication 1, **caractérisé en ce que** les éléments en forme de barrettes (2) sont déformés de telle sorte que la déformation, à laquelle est soumis globalement un élément en forme de barrette entre des zones de liaison avec d'autres éléments en forme de barrettes (2), est essentiellement réduite.

10. Extenseur selon la revendication 1, **caractérisé en ce que** les différents éléments en forme de barrettes (2) sont conformés de telle sorte que la déformation de coudage, à laquelle est soumis un élément en forme de barrette (2) lors de l'extension, à l'intérieur de la fente tubulaire cylindrique creuse, et qui résulte de l'intégrale des variations angulaires locales lors de la déformation, est déterminée sur la longueur de l'élément en forme de barrette respectif entre des zones de liaison contiguës (5) avec d'autres éléments en forme de barrettes (2), répartis sur la longueur de l'élément de sorte qu'elle ne dépasse pas localement une valeur prédéterminée.

11. Barrette selon la revendication 1, **caractérisée en ce que** la déformation de coudage, qui se produit sur une longueur égale au cinquième d'un élément en forme de barrette (6), n'est pas supérieure à un quart de la déformation totale par coudage, à laquelle est soumis cet élément en forme de barrette (2).

12. Extenseur selon la revendication 1, **caractérisé en ce que** la forme de l'extenseur (1) à l'état non déployé correspond essentiellement à la forme que l'on obtient lorsqu'un modèle, possédant à l'état déployé des structures en forme de barrettes ayant une forme uniforme et établi dans cette forme à partir d'une structure de forme tubulaire, est comprimé sous la forme non déployée (la forme de départ ultérieure).

13. Extenseur selon la revendication 12, **caractérisé en ce que** la forme régulière est constituée par des cercles, des ellipses, des rectangles, des carrés, des polygones ou par des structures composées de telles formes ou s'en rapprochant.

14. Extenseur selon la revendication 1, **caractérisé en ce que** des ramifications de barrettes sont formées tout en évitant des variations brusques de la largeur des barrettes.

15. Extenseur selon la revendication 1, **caractérisé en ce que** les ramifications sont agencées de telle sorte que les contraintes du matériau ne dépassent pas, notamment dans la zone de la ramification, lors de la déformation, également en tant que tension d'encoche, une valeur prédéterminée.

16. Extenseur selon la revendication 1, **caractérisé en ce que** les éléments en forme de barrettes (11') sont agencés essentiellement avec une forme en S.

17. Extenseur selon la revendication 1, **caractérisé par** du titane, du tantale ou un autre métal biocompatible ou un alliage métallique correspondant en tant que matériau.

18. Extenseur selon la revendication 17, **caractérisé en ce qu'**il est prévu un revêtement formé de carbure de silicium amorphe.
